# EUROPÄISCHE PATENTSCHRIFT

(11) **EP 2 548 598 B1**
(45) Veröffentlichungstag und Bekanntmachung des Hinweises auf die Patenterteilung: **07.01.2015**
(21) Anmeldenummer: 12189004.0
(22) Anmeldetag: 06.05.2009
(51) Int. Cl.: A61M 11/00, A61M 15/00, A61M 16/10, A61M 16/08

(54) **Vernebler für Beatmungsmaschinen und Beatmungsmaschine mit einem solchen Vernebler**
Atomiser for respiration machines and respiration machine with such an atomiser
Nébuliseur pour respirateur et respirateur équipé d'un tel nébuliseur

(30) Priorität: 09.05.2008 DE 102008022987
(43) Veröffentlichungstag der Anmeldung: 23.01.2013
(62) Teilanmeldung aus: 09742089.7
(73) Patentinhaber: PARI Pharma GmbH, 82319 Starnberg (DE)
(72) Erfinder: Gallem, Thomas, 81371 München (DE); Hetzer, Uwe, 81369 München (DE)
(74) Vertreter: Hoffmann Eitle

(56) Entgegenhaltungen:
- WO-A-2005/048982
- DE-A1-102005 038 619
- US-A- 5 474 058
- US-A1- 2003 072 717
- US-A1- 2006 289 002
- US-A1- 2008 000 470

## Beschreibung

Die vorliegende Erfindung betrifft Vernebler für Beatmungsmaschinen und insbesondere Vernebler zum Einbringen eines Wirkstoffs in Aerosolform in die zur unterstützenden oder ersetzenden Beatmung einem Patienten zugeführte Beatmungsluft. Hierbei ist unter einem Vernebler eine beliebige Vorrichtung zur Erzeugung eines Aerosols zu verstehen. Unter dem Begriff einer Beatmungsmaschine ist ein Beatmungsgerät zu verstehen, dass mit einem Endotrachealtubus intubierte oder tracheotomierte Patienten mit einen Luftfluss von bis zu 120 l/min, vorzugsweise zwischen 1 und 50 l/min bei einem Druck zwischen 0 und 100 mbar und vorzugsweise zwischen 3 und 45 mbarversorgt werden.

Des Weiteren betrifft die vorliegende Erfindung auch eine Beatmungsmaschine mit einem derartigen Vernebler.

Vernebler für Beatmungsmaschinen sind im Stand der Technik gut bekannt. So offenbart beispielsweise die WO 2005/048982 A2 einen Vernebler. Dieser Vernebler umfasst einen Körper mit einem ersten Anschluss, der zwei Anschlussstutzen umfasst, um den Vernebler mit einer Zuluft- und einer Abluftleitung des Beatmungsgeräts zu verbinden. Entgegengesetzt des ersten Anschlusses weist der Körper einen zweiten Anschluss wiederum mit zwei Anschlussstutzen auf, die über ein Y-Stück und zwei Schläuche mit einer Leitung zum Patienten zu verbinden sind. Innerhalb des Körpers sind zwei getrennte über einen Verbindungskanal verbundene Strömungskanäle gebildet, von denen einer (erster) der Strömung der Beatmungsluft aus der Zuluftleitung zum Patienten und der andere (zweiter) der Strömung der verbrauchten Luft vom Patienten in die Abluftleitung dient. Stromaufwärts einer Verbindung einer Vernebelungseinrichtung mit dem ersten Strömungskanal ist ein Rückschlagventil in dem ersten Strömungskanal angeordnet, das eine Strömung ausschließlich in Richtung des Patienten gestattet. Darüber hinaus ist die Vernebelungseinrichtung senkrecht, ähnlich einer T-Verbindung, mit dem ersten Strömungskanal gekoppelt, wobei das Aerosol in einer Richtung senkrecht zur Strömungsrichtung in diesem ersten Strömungskanal zugeführt wird.

Bei dieser Ausgestaltung ergibt sich zum einen die Problematik, dass mit dem Rückschlagventil ein Element in die Zuluftleitung, d. h. in die Leitung, die zum Patienten führt, integriert wird, welches bei einer Funktionsstörung schwerwiegende Folgen haben kann.

Zum anderen besteht die Problematik, dass das Aerosol senkrecht zur Strömungsrichtung der Beatmungsluft durch den Körper in die Strömung eingeführt wird und damit eine hohe Deposition des Aerosols an den Oberflächen des Strömungskanals mit einem damit verbundenen hohen Verlust auftritt.

Aus anderen Gebieten der Technik ist es zwar bekannt die Deposition des Aerosols an Oberflächen insbesondere der Vernebelungskammer dadurch zu verhindern, dass die Verneblungseinrichtung in einer Richtung vernebelt, die parallel zu einer Strömung zum Patienten verläuft. Beispielsweise offenbart die US-A-2003/0072717 eine Inhalationsvorrichtung bei der eine Verneblungseinrichtung in einem geschlossenen und umströmten Gehäuse angeordnet ist. Das Gehäuse sitzt in einem Strömungskanal des Inhalators, der ein Mundstück aufweist. Die Verneblungseinrichtung vernebelt dabei in Richtung des Mundstücks. Hier spielen jedoch Totraumvolumen, Strömungswiderstand durch die Verneblungseinrichtung sowie deren Befüllung nur untergeordnete Rollen. Bei Beatmungsmaschinen müssen die Venebler jedoch diesbezüglich vorgegebene Kriterien erfüllen. Auch die gattungsbildende EP-A- 1 818 070 (US-A-2008/000470) offenbart eine Inhalationstherapievorrichtung mit einer derartigen Vernebelungsrichtung, hier jedoch für Frühchen. Das System (setzt eine selbstständige Atmung des Patienten voraus und) ist speziell auf die kleinen Leitungsquerschnitte zwischen 2 mm bis 3,5 mm Innendurchmesser für Frühchen ausgerichtet, so dass eine Umströmung der Vernebelungseinrichtung leicht realisierbar ist, ohne dass ein Strömungswiderstand entsteht. Ferner operiert das System auf Grund der selbständigen Atmung des Patienten bei niedrigem Druck von bis zu 15 mbar. Auch ist ein Befüllen des Verneblers mit zu vernebelndem Fluid nicht während des Betriebs des Systems nötig oder vorgesehen, weil der Betrieb zum Befüllen unterbrochen werden kann und/oder ein Druckverlust im System gefahrlos in Kauf genommen werden kann. Daher waren oben beschriebene und vergleichbare Systeme aus dem Stand der Technik nicht auf den Einsatz in Beatmungsmaschinen übertragbar.

Ferner beschreibt die DE-A-10 2005 038 619 A1 eine Inhalationstherapievorrichtung mit einer Ampulle für die Bereitstellung eines zu vernebelnden Medikaments, welche über einen Dorn geöffnet werden kann. Beim Öffnen wird das zu vernebelnde Medikament der Verneblungseinrichtung, hier einer Membran, zugeführt.

Darüber hinaus ist aus der US-A-2006/0289002 ein Vernebler für Beatmungsmaschinen bekannt, bei dem die Beatmungsluft parallel zu einer Membran der Verneblungseinrichtung eingespeist und zur Ausbildung einer Hüllströmung umgelenkt wird. Die Hüllströmung dient der Vermeidung von Aerosoldepositionen an der Innenwandung des Systems.

Die Aufgabe der vorliegenden Erfindung liegt folglich darin einen Vernebler für Beatmungsmaschinen zu schaffen, bei dem die Deposition von Aerosol an den Oberflächen des Strömungskanals und die damit verbundenen Verluste reduziert sind sowie auf Fehler anfällige Elemente verzichtet werden kann. Gleichzeitig darf jedoch die Funktionsweise der Beatmungsmaschine durch den Vernebler nicht beeinträchtig sein, d. h. es darf kein Strömungswiderstand erzeugt werden, der Vernebler muss bei einem gewissen Überdruck (z. B. 100 mbar) dicht sein, eine Befüllung des Verneblers mit dem zu vernebelnden Fluid muss auch während des Betriebs des Beatmungsgeräts ohne Druckverlust im System möglich sein, und ein Totraum in dem Vernebler darf kein zu großes Volumen annehmen etc.

Diese Aufgabe wird durch einen Vernebler mit den Merkmalen des Patentanspruchs 1 gelöst. Vorteilhafte Weiterbildungen der vorliegenden Erfindung sind in den Unteransprüchen genannt.

Der vorliegenden Erfindung liegt der Gedanke zu Grunde, die Verneblungseinrichtung in den Strömungskanal zu verlegen, so dass die Aerosolerzeugung durch Vernebeln des Fluids parallel zur Strömungsrichtung im Strömungskanal erfolgt, statt die Verneblungseinrichtung senkrecht zur Strömungsrichtung über eine T-Stück artige Anordnung zu koppeln, wie es im Stand der Technik der Fall ist.

Die vorliegende Erfindung schlägt dementsprechend einen Vernebler für Beatmungsmaschinen vor, der einen Körper mit einem ersten Anschluss zur Verbindung (z.B. indirekten Verbindung über einen Schlauch) des Verneblers mit einem Beatmungsgerät und einem zweiten Anschluss zur Verbindung (z.B. indirekten Verbindung über einen Schlauch)des Verneblers mit einer zum Patienten führenden Leitung umfasst, wobei der Körper einen, insbesondere nur einen, Strömungskanal von dem ersten Anschluss zum zweiten Anschluss bildet. D. h. in einer besonderen Ausführungsform bildet der Körper nur einen Strömungskanal, durch den beim Einatmen Beatmungsluft vom ersten Anschluss zum zweiten Anschluss und beim Ausatmen verbrauchte Luft vom zweiten Anschluss zum ersten Anschluss strömt. Die Leitung, die zum Patienten führt kann sich aus der sog. Gänsegurgel (Doppeldrehkonnektor) und dem Endotrachealtubus sowie ggf. weiteren Elementen zusammensetzen. Die Verbindung mit dem Beatmungsgerät kann bei einer besonderen Ausführungsform sowohl mit der Zuluftleitung zur Zufuhr von Beatmungsluft als auch der Abluftleitung zum Ausgeben von verbrauchter Luft erfolgen, so dass der Vernebler gleichzeitig ein Y-Stück bildet. Es versteht sich jedoch, dass der erste Anschluss des Körpers auch nur mit der Zuluftleitung verbunden sein kann, so dass ein etwaiges Y-Stück erst dem zweiten Anschluss nachgeschaltet angeordnet wird. Ferner weist der Vernebler der vorliegenden Erfindung eine Vernebelungseinrichtung zur Vernebelung eines Fluids auf. Bei dem Fluid handelt es sich vorzugsweise um eine flüssige Zusammensetzung, die vorzugsweise wenigstens einen Wirkstoff (siehe später) enthält. Unter einer Vernebelungseinrichtung ist dabei jeglicher Aerosolgenerator bzw. -erzeuger zu verstehen, über den sich das Fluid in Aerosolform bringen lässt. Erfindungsgemäß kennzeichnet sich der Vernebler dadurch, dass die Vernebelungseinrichtung zwischen dem ersten Anschluss und dem zweiten Anschluss in dem Strömungskanal angeordnet ist und so ausgestaltet ist, dass das Fluid im Wesentlichen parallel zur, vorzugsweise in, Strömungsrichtung vom ersten Anschluss zum zweiten Anschluss vernebelbar ist. Mit anderen Worten wird die Vernebelungseinrichtung im Einatemzyklus von Beatmungsluft umströmt und das zu vernebelnde Fluid parallel zur, vorzugsweise in Richtung der Beatmungsluftströmung, vernebelt, so dass eine Aerosolströmung parallel zur, vorzugsweise in Strömungsrichtung der ersten Beatmungsluft erzeugt wird, wodurch die Impaktion und damit Deposition an Oberflächen im Strömungskanal vermindert wird und das Aerosol kann möglichst verlustfrei in das Leitungssystem eingespeist und zum Patienten transportiert werden. Die Terminologie "im Wesentlichen" ist diesbezüglich derart zu verstehen, dass die Aerosolströmung auch mit einer Abweichung von bis zu 45° zur Strömungsrichtung der Beatmungsluft verlaufen kann.

Zur Regulierung der Beatmungsluft erzeugen Beatmungsgeräte eine kontinuierliche Grundströmung (einen sog. "bias flow"). Außerhalb eines Einatemzyklus, in dem Beatmungsluft über die Zuluftleitung zum Patienten gefördert wird, strömt diese Grundströmung, die in einer Größenordnung von bis zu 30 l/min liegen kann, üblicherweise von der Zuluftleitung direkt in die Abluftleitung. Um dies zu ermöglichen, ohne dass die Grundströmung die Vernebelungseinrichtung passiert und damit permanent vernebeltes Fluid in die Abluftleitung strömt, was zu einer erheblichen Ineffizienz des Systems führen würde, umfasst der Vernebler der vorliegenden Erfindung vorzugsweise einen ersten Anschluss, der derart zur Verbindung mit einer Zuluftleitung von dem Beatmungsgerät und einer Abluftleitung zu dem Beatmungsgerät gestaltet ist, dass auf der dem zweiten Anschluss entgegengesetzten Seite der Verneblungseinrichtung ein Nebenströmungskanal (Bypass) von der Zuluftleitung in die Abluftleitung gebildet ist.

Dabei können die Zuluftleitung und die Abluftleitung durch einen gemeinsamen, in zwei Abschnitte unterteilten Schlauch gebildet sein. Diese Unterteilung kann entweder durch eine Trennwand in dem Schlauch oder aber durch einen Koaxialschlauch, d. h. zwei ineinander angeordnete Schläuche gebildet sein. Der gemeinsame Schlauch ist mit dem ersten Anschluss des Verneblers zu verbinden. Der erste Anschluss sowie der gemeinsame Schlauch sind dabei derart ausgestaltet, dass zwischen der Verneblungseinrichtung und der der Verneblungseinrichtung zugewandte Stirnseite der Trennwand und/oder des inneren Schlauchs des Koaxialschlauchs ein Nebenströmungskanal gebildet ist. Der Nebenströmungskanal ermöglicht, dass außerhalb eines Einatemzyklus die Grundströmung von der Zuluftleitung direkt in die Abluftleitung strömen kann ohne ggf. vernebeltes Fluid in die Abluftleitung zu spülen. Dadurch kann die Effizienz des Systems erheblich verbessert werden.

Alternativ ist es selbstverständlich auch denkbar, dass die Zuluft- und die Abluftleitung jeweils durch einen separaten Schlauch gebildet sind. Bei dieser Ausgestaltung ist es bevorzugt, dass der erste Anschluss einen ersten Anschlussstutzen zur Verbindung mit der Zuluftleitung und einen zweiten Anschlussstutzen zur Verbindung mit der Abluftleitung, die jeweils durch einen Schlauch gebildet sein können, aufweist. Der Nebenströmungskanal ist dann zwischen dem ersten Anschlussstutzen und dem zweiten Anschlussstutzen im Körper selbst ausgebildet und ermöglicht außerhalb des Einatemzyklus die Strömung aus der Zuluftleitung in die Abluftleitung. Diese Ausgestaltung führt ferner dazu, dass durch die zwei Anschlussstutzen sowie den gegenüberliegenden zweiten Anschluss eine kippstabile Einheit des Verneblers gebildet wird.

Wie es bereits eingangs erwähnt wurde, muss der Vernebler die Befüllung eines Fluidbehälters auch während der Beatmung ohne Druckverlust im System gestatten. Es wird daher als Verneblungseinrichtung eine vibrierbare Membran (Schwingmembran) verwendet, die zur Verneblung des Fluids mit einer Vielzahl von Öffnungen (Mikroöffnungen) versehen ist. Die Membran ist zur Erzielung der Verneblung bevorzugt in Strömungsrichtung bzw. parallel zur Strömungsrichtung senkrecht zur Strömungsrichtung vom ersten Anschluss zum zweiten Anschluss angeordnet ist. Die Terminologie "im Wesentlichen" ist diesbezüglich derart zu verstehen, dass die Membran auch in einer leichten Neigung von bis zu 45° Abweichung von der Senkrechten in dem Strömungskanal angeordnet sein kann. Die Membran ist dabei aus strömungstechnischen Gesichtspunkten bevorzugter Weise kreisrund ausgestaltet, kann aber auch oval sein. Durch diese Ausgestaltung des Aerosolerzeugers (Vernebelungseinrichtung) mit einer sehr kleine Öffnungen aufweisenden Membran, durch die eine Strömung aus dem Strömungskanal und aus dem System nicht möglich ist, wird auf einfachste Weise garantiert, dass ein Druckverlust über die Verneblungseinrichtung verhindert wird, auch wenn der Fluidbehälter geöffnet, z.B. der Deckel des Fluidbehälters zum Befüllen abgeschraubt wird.

Darüber hinaus ist ein mit dem Körper verbundener Fluidbehälter zur Aufnahme des zu vernebelnden Fluids vorgesehen sowie eine Verneblungskammer, in die das Fluid zu vernebeln ist, wobei die Membran zwischen dem Fluidbehälter und der Vernebelungskammer angeordnet ist. Dabei ist der Fluidbehälter an- und abkoppelbar mit dem Körper verbunden. Um die Deposition und damit den Verlust an den Oberflächen des Strömungskanals weiter zu minimieren, ist es, wie erwähnt, besonders bevorzugt das Fluid im Einatemzyklus in Strömungsrichtung der Beatmungsluft zu vernebeln, wozu die Verneblungskammer vorzugsweise zwischen der Membran und dem zweiten Anschluss, der mit der Leitung zum Patienten zu binden ist, liegt.

Darüber hinaus ist es erforderlich, dass der Fluidbehälter bis zu einer Neigung von 45° in jede Richtung um die Strömungsrichtung vom ersten zum zweiten Anschluss eine konsistente Dosis zur Verneblermembran führt, so dass eine zuverlässige und gleichmäßige Verneblung bzw. Aerosolerzeugung stattfinden kann. Zu diesem Zweck kann einerseits, wie bereits erwähnt die Einheit aus Körper und Fluidbehälter durch die zwei Anschlussstutzen und den entgegengesetzten zweiten Anschluss kippstabil ausgestaltet werden. Um diese Anforderung weiter zu erfüllen, ist es jedoch bevorzugt, dass der Fluidbehälter eine Verjüngung zur Membran hin umfasst, die in eine durch die Membran abgeschlossene Fluidkammer mündet, wobei sich die Verjüngung zumindest schräg von einem zylindrischen Abschnitt des Fluidbehälters zu der Fluidkammer erstrecken sollte.

Besonders bevorzugt ist es, wenn sich ein der Membran abgewandter Teilbereich der Verjüngung in einem Winkelbereich zwischen 10° und 40° zur Vertikalen und bei senkrecht angeordneter Membran vorzugsweise auch zur Membran erstreckt, d. h. ein unterer Bereich des im Querschnitt geschlossenen Fluidbehälters ist z.B. konusförmig ausgestaltet und die Mittelachse des Konus liegt in einem Winkelbereich zwischen 10° und 30° zur Vertikalen und bei senkrecht angeordneter Membran vorzugsweise auch zur Membran .

Wie es bereits eingangs erwähnt wurde, sind die Verneblungseinrichtung und insbesondere die Membran in dem Strömungskanal so angeordnet, dass sie umströmt wird. Dieser Umströmungsabschnitt des Strömungskanals wird vorzugsweise in Radialrichtung zwischen der Membran und dem Körper derart ausgebildet, dass eine Querschnittsfläche des Strömungsabschnitts im Wesentlichen der kleinsten Querschnittsfläche einer Leitung des Beatmungsgeräts zum Patienten entspricht, obwohl kleinere Abweichungen möglich sind. Bevorzugt ist die Querschnittsfläche des Strömungsabschnitts größer als der kleinste Querschnitt einer Leitung, wenigstens jedoch zumindest nahezu gleich. Der Querschnittbereich liegt dabei für Erwachsene in einem Bereich von ca. 400 mm². Bei Kleinkindern liegt der Querschnittsbereich in einem Bereich von ca. 80 - 180 mm². Dadurch wird auf einfachste Art verhindert, dass der Strömungswiderstand durch Integration des Verneblers in die Zuluft- und Patientenleitung zu stark erhöht wird und zu einer Funktionsbeeinträchtigung des Beatmungsgeräts bzw. der Beatmungsmaschine führen könnte.

Besonders bevorzugt, ist es die Membran über Stege in einem die Membran umgebenden Rahmen aufzuhängen, wobei der Rahmen vorzugsweise aus strömungstechnischen Gründen ebenfalls kreisrund oder oval, bevorzugterweise in der gleichen Ausgestaltung, wie die Membran ausgestaltet ist. Zwischen dem Rahmen und der Membran wird damit ebenfalls ein durchströmbarer Bereich gebildet, der zumindest einen Teil des Umströmungsabschnitts des Strömungskanals bilden kann. Ggf. können zwischen dem Rahmen und dem Körper weitere durchströmbare Abschnitte vorgesehen sein, die den zwischen Membran und Rahmen vorhandenen Teil des Umströmungsabschnitts ergänzen können, um die gewünschte Querschnittsfläche für den Umströmungsabschnitt zu erzielen. Ferner kann durch diese Ausgestaltung der durch die Verneblungskammer entstehende Totraum vermindert und gleichzeitig der Querschnitt des Strömungskanals vorbei an der Vernebelungseinrichtung erhöht werden, ohne dass die äußeren Dimensionen des Verneblers wesentlich zunehmen.

Um die Effizienz des Systems weiter zu erhöhen, kann es bevorzugt sein den Vernebler über eine gemeinsame oder zusammenwirkende Steuerung mit dem Beamtungsgerät anzusteuern, so dass eine Verneblung des Fluids und damit eine Aerosolerzeugung nur während des Einatemzyklus auslösbar ist, d. h. eine Verneblung durch die Verneblungseinrichtung findet nur dann statt, wenn der Patient einatmet, sei dies unterstützt oder erzwungen durch das Beatmungsgerät.

Weitere Vorteile und Merkmale der vorliegenden Erfindung, die alleinstehend oder in Kombination mit den obigen Merkmalen kombinierbar sind, sind aus der folgenden Beschreibung bevorzugter Ausführungsformen ersichtlich. Diese Beschreibung erfolgt unter Bezugnahme auf die begleitenden Zeichnungen, in denen:
Fig. 1 eine perspektivische Ansicht eines Verneblers gemäß einer ersten Ausführungsform, die nicht Teil der beanspruchten Erfindung ist, schematisch angekoppelt an ein Beatmungsgerät zeigt;
Fig. 2 eine Draufsicht auf den Vernebler in Fig. 1 zeigt;
Fig. 3 eine Seitenansicht auf den Vernebler in Fig. 1 auf dem Kopf stehend zeigt;
Fig. 4 einen Längsschnitt durch den Vernebler aus Fig. 1 entlang der Linie A-A in Fig. 2 zeigt;
Fig. 5 einen Längsschnitt des Verneblers aus Fig. 1 entlang der Linie B-B in Fig. 3 zeigt;
Fig. 6a-f, (a) einen Längsschnitt durch den Vernebler aus Fig. 1 entlang der Linie A-A in Fig. 2 und (b) einen Längsschnitt des Verneblers aus Fig. 1 entlang der Linie B-B in Fig. 3 mit einer Fluidströmung während des Einatemzyklus zeigen und (c)-(d) die gleichen Schnitte während des Ausatemzyklus zeigen sowie (e)-(f) die gleichen Schnitte zwischen dem Ausatemzyklus und dem Einatemzyklus zeigen,
Fig. 7 einen Längsschnitt durch einen Vernebler gemäß einer zweiten Ausführungsform, die nicht Teil der beanspruchten Erfindung ist, entsprechend dem Schnitt aus Fig. 4 zeigt; und
Fig. 8 schematisch einen alternativen Fluidbehälter gemäß der Erfindung zeigt

In den verschiedenartigen Ansichten sind gleiche oder entsprechende Elemente mit den gleichen Bezugsziffern versehen.

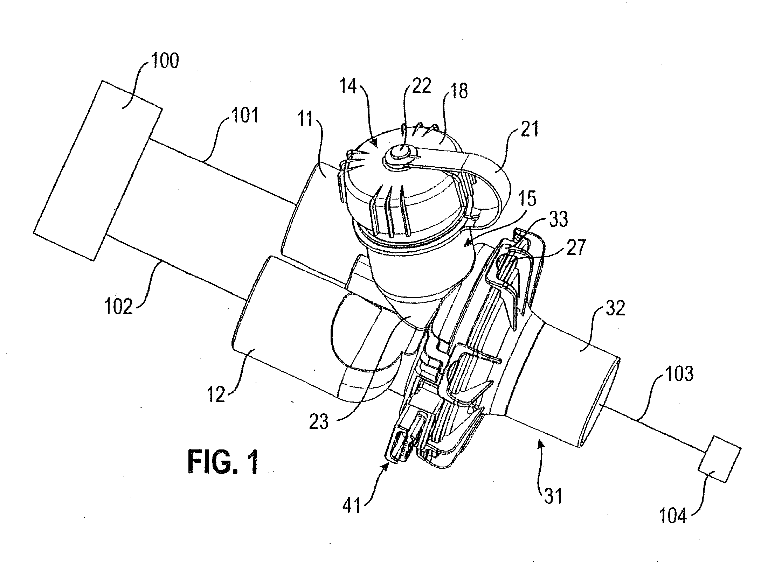

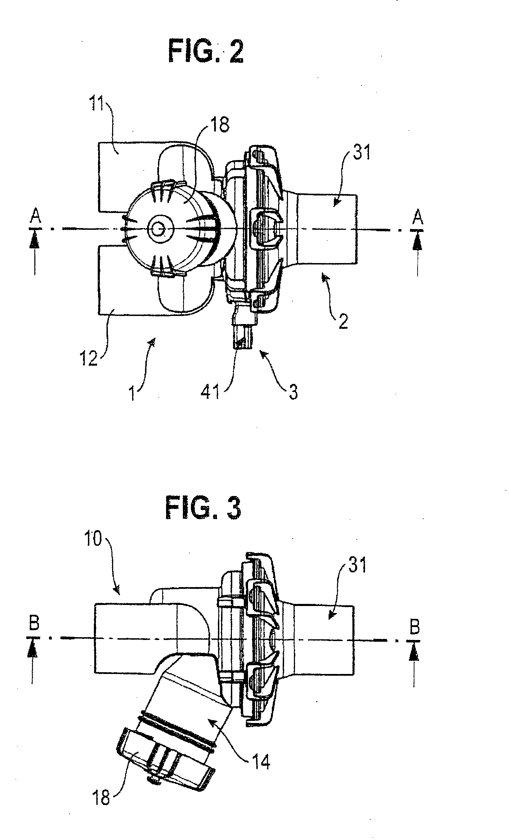

Der Vernebler der ersten Ausführungsform, der in den Fig. 1-5 dargestellt ist, setzt sich aus drei Hauptkomponenten, einem ersten Körperabschnitt 1, einem zweiten Körperabschnitt 2 sowie einer Verneblungseinrichtung 3 zusammen. Der erste und zweite Körperteil 1 und 2, die zusammen den Körper bilden, sind vorzugsweise aus Kunststoff und vorzugsweise in einem Spritzgießverfahren hergestellt

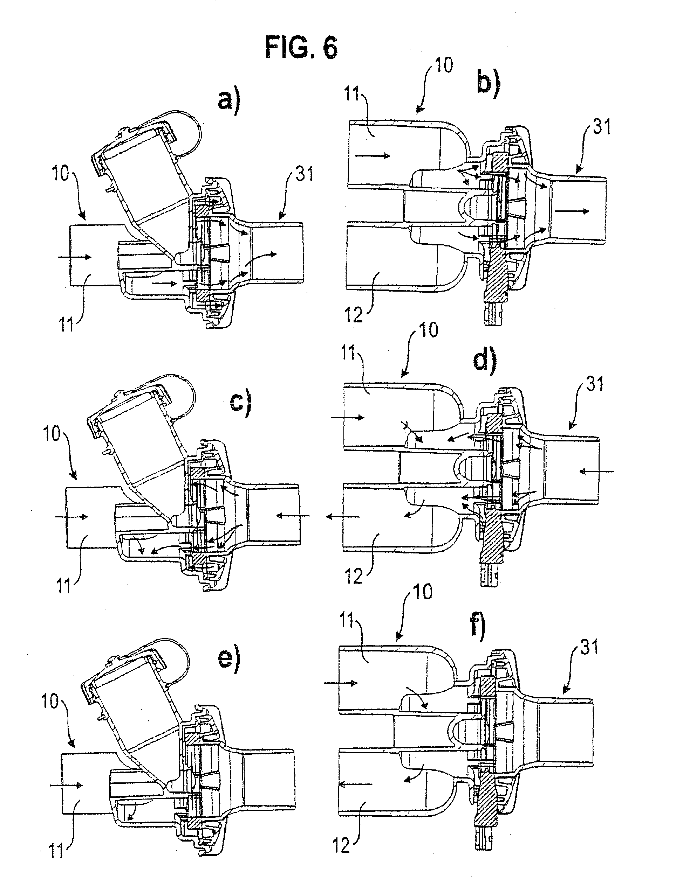

Der erste Körperteil 1 umfasst einen ersten Anschluss 10, der sich aus zwei Anschlussstutzen 11, 12 zusammensetzt. Wie es aus Fig. 1 ersichtlich ist, ist der erste Anschlussstutzen 11 zur Verbindung mit einer Zuluftleitung des Beatmungsgeräts 100 ausgestaltet. Der zweite Anschlussstutzen 12 wiederum ist mit einer Abluftleitung 102 des Beatmungsgeräts 100 gekoppelt. Dabei sind die Zuluftleitung 101 und die Abluftleitung 102 jeweils durch einen separaten Schlauch (nicht dargestellt) gebildet, der beispielweise einen Innendurchmesser von 22 mm für Erwachsene oder einen Innendurchmesser von 10 mm und 15 mm für Kinder aufweisen kann. Die Anschlussstutzen 11, 12 sind jeweils derart gestaltet, dass ein Kopplung dieser herkömmlichen Schläuchen mit den Anschlussstutzen möglich ist. Des Weiteren ist in dem ersten Körperteil 1 ein Nebenströmungskanal 13 ausgebildet, der vor (d. h. in Strömungsrichtung der Beatmungsluft stromauf) der Vernebelungseinrichtung 3 angeordnet ist. Dieser Nebenströmungskanal dient dazu, dass eine Grundströmung, die von dem Beatmungsgerät 100 zur Regulierung der Beatmungsluft zu einem Patienten 104 erzeugt wird außerhalb eines Einatemzyklus und/oder eines Ausatemzyklus durch den Patienten 104 direkt von der Zuluftleitung 101 über den Anschlussstutzen 11, den Nebenströmungskanal 13, den Anschlussstutzen 12 in die Abluftleitung 102 strömen kann, ohne dass die Verneblungseinrichtung 3 passiert wird (siehe Fig. 6e und f). Bei dieser Grundströmung handelt es sich um einen Durchfluss von bis zu 30 l/min. Diese Grundströmung wird oftmals auch als "bias flow" bezeichnet.

Des Weiteren umfasst der erste Körperteil 1 auch einen Fluidbehälter 14 zur Aufnahme eines zu vernebelnden Fluids. Mögliche Fluide, die bei der vorliegenden Ausführungsform vorzugsweise in flüssiger Form vorliegen, sind im Anschluss an die Beschreibung der bevorzugten Ausführungsformen aufgelistet. Der Fluidbehälter 14 ist hier integraler Bestandteil des ersten Körperteils 1, ist erfindungsgemäß jedoch vollständig oder teilweise ab- und ankoppelbar ausgestaltet

Gemäß der dargestellten Ausführungsform umfasst der Fluidbehälter 14 jedoch einen im Wesentlichen zylindrischen Abschnitt 15 mit im Wesentlichen kreisrunden Querschnitt. An dem der Verneblungseinrichtung 3 abgewandten Ende des zylindrischen Abschnitts 15 ist ein Außengewinde 16 an der Außenumfangsfläche des zylindrischen Abschnitts 15 ausgebildet. Mit diesem Außengewinde 16 ist ein Innengewinde 17 eines Deckels 18, das an der Innenumfangsfläche des Deckels 18 ausgebildet ist, in Eingriff bringbar, um den Deckel 18 auf den zylindrischen Abschnitt 15 des Fluidbehälters 14 aufschrauben zu können. Ferner umfasst der Deckel an seiner Innenseite einen umlaufenden Kragen 19, der beim Verschrauben des Deckels 18 mit der Innenfläche des zylindrischen Abschnitts 14 direkt oder über ein Dichtungsmaterial indirekt abdichtend in Eingriff kommt. Des Weiteren umfasst der zylindrische Abschnitt 15 eine umlaufende Nut 20, in der ein Ende einer Deckelsicherung 21 (siehe Fig. 1) befestigbar ist, die anderen Endes an dem pilzkopfförmigen Vorsprung 22 des Deckels 8 anbringbar ist.

An das, dem Deckel abgewandte Ende des zylindrischen Abschnitts 15 schließt sich ein verjüngender Abschnitt 23 an, der sich zur Verneblungseinrichtung 3 hin verjüngt und in eine Fluidkammer 24 mündet. Der verjüngte Abschnitt 23 setzt sich bei der dargestellten Ausführungsform im Querschnitt aus einer im Wesentlichen parallel zum Verlauf der später beschriebenen Membran 37 erstreckenden Wandung 26 sowie einer sich in einem Winkel zwischen 40 und 50° zur Vertikalen bzw. zur Membran 37 verlaufenden Wandung 25 zusammen und ist im Wesentlichen konisch ausgebildet. Der Scheitelpunkt des Konus liegt dabei im Wesentlichen in der Fluidkammer 24.

Ein mit Fluid F befüllter Fluidbehälter 14 ist beispielsweise in Fig.1 sichtbar.

Des Weiteren umfasst der erste Körperteil 1 an seinem dem ersten Anschluss 10 entgegengesetzten Ende einen umlaufenden Kragen 27, der mit dem zweiten Körperteil 2 (siehe später) koppelbar ist, auf. Radial innerhalb dieses Kragens 27 ist ein Dichtungsmaterial 28 angespritzt bzw. im ZweiKomponenten-Verfahren mit dem aus hart elastischem Kunststoff bestehenden ersten Körperteil 1 hergestellt. Dieses Dichtungsmaterial 28 weist einen umlaufenden Vorsprung 29 auf. Ferner ist eine sich an die Fluidkammer 24 anschließende umlaufende Dichtlippe 30, die zur Abdichtung gegen die Membran 37 gedrückt wird, so dass die Fluidkammer 24 durch die Membran 37 und die Dichtlippe 30 dicht abgeschlossen wird, vorgesehen.

Der zweite Körperteil 2 umfasst den zweiten Anschluss 31, der aus einem Anschlussstutzen 32 gebildet ist. Dieser Anschlussstutzen 32 ist vorzugsweise gleichermaßen ausgestaltet wie der mit den Anschlussstutzen 11 und 12 jeweils zu verbindende Schlauch, der die Leitungen 101 bzw. 102 bildet. Dadurch kann gewährleistet werden, dass der dargestellte Vernebler nur sachgemäß in dem Beatmungsgerät integriert werden kann. Auch andere Ausgestaltungen um dies zu erzielen, sind denkbar. So ist es lediglich wesentlich, dass die Anschlüsse 31 und 10 nicht identisch ausgestaltet sind, so dass eine Verbindung einer der Leitungen 101, 102, die durch jeweils Schläuche gebildet sind, mit dem Anschlussstutzen 32 oder aber eine Verbindung der zweiten Leitung 103, die zum Patienten 104 führt, mit einem der Anschlussstutzen 11 oder 12 ausgeschlossen ist.

Des Weiteren umfasst der zweite Körperteil 2 über seinen Umfang verteilt mehrere Verriegelungseinrichtungen, hier Rastnasen 33. Bei der dargestellten Ausführungsform sind sechs derartige Verriegelungsnasen 33 bzw. Schnapphaken vorgesehen. Es sind jedoch auch weniger oder mehr derartige Einrichtungen denkbar. Dabei sind die Verriegelungsnasen 33 derart gestaltet, dass sie im montierten Zustand mit dem umlaufenden Kragen 27 des ersten Körperteils 1 in Eingriff gebracht werden können, indem sie den Kragen 27 hintergreifen, so dass das erste und zweite Körperteil 1 bzw. 2 miteinander verbindbar sind. Ferner weist der zweite Körperteil 2 radial innerhalb der Verriegelungseinrichtungen 33 zwei umlaufende konzentrisch angeordnete Stege 34 und 35 auf, die in ihrem Abstand in Radialrichtung an die Breite des Vorsprungs 29 des Dichtmaterials 28 in Radialrichtung derart angepasst sind, dass beim Eingriff des ersten und zweiten Körperteils 1 bzw. 2 zwischen dem Vorsprung 29 und den beiden Stegen 34 und 35 eine labyrinthartige Dichtung entsteht.

Des Weiteren umfasst der zweite Körperteil 2 wenigstens zwei, vorzugsweise vier und ggf. mehr Stützvorsprünge 36 zum Halten des Aerosolgenerators 3 (Verneblungseinrichtung) (siehe später). Diese sind gleichmäßig über den Umfang des zweiten Verriegelungskörpers 2 jeweils paarweise diametral gegenüberliegend und bei vier Elementen jeweils um 90° versetzt angeordnet.

Der zweite Körper kann rotationssymmetrisch ausgestaltet sein, so dass er in einer beliebigen Ausrichtung um seine Mittelachse mit dem ersten Körperteil 1 verbunden werden kann.

Die Vernebelungseinrichtung 3 umfasst eine Membran 37, die eine Vielzahl winziger Öffnungen bzw. Löcher im Mikrobereich umfasst, die die Membran vollständig durchdringen. Die Membran 37 ist vorzugsweise über ein piezoelektrisches Element vibrierbar, d. h. sie kann in Schwingung versetzt werden. Durch die Schwingung der Membran wird Flüssigkeit auf einer Seite der Membran, d. h. aus der Fluidkammer 24 durch die Öffnungen (nicht sichtbar) treten und auf der anderen Seite der Membran 37 in eine in dem Körper gebildete Verneblungskammer 38 vernebelt. Dieses allgemeine Prinzip ist z. B. in der US 5,518,179 genauer erläutert, so dass auf eine detaillierte Beschreibung dieser Funktionsweise hier verzichtet wird.

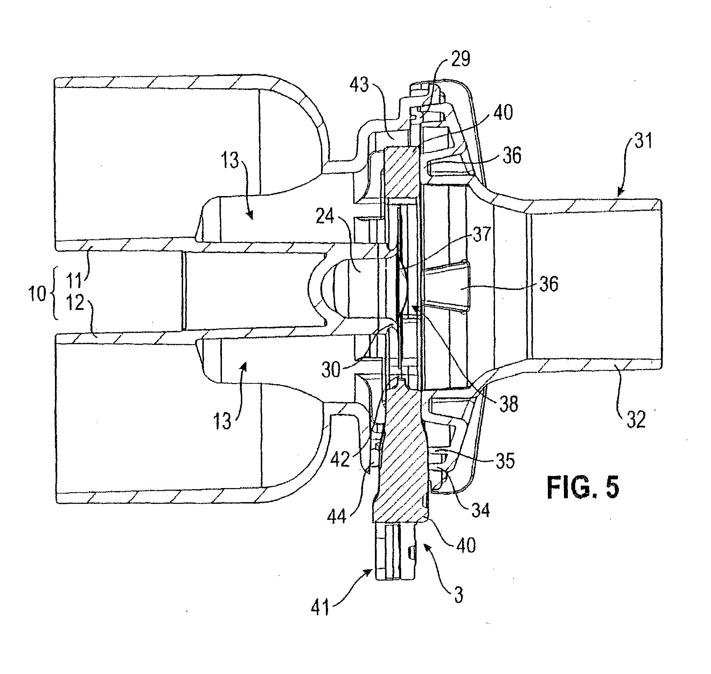

Erfindungsgemäß wird die Membran 37, bei der es sich um ein flächiges ebenes Element handelt, über (in den Zeichnungen nicht sichtbare) Stege in einem (nicht sichtbaren) Rahmen gehalten. Die Membran 37 wie auch der Rahmen sind im Wesentlichen kreisrund bzw. ringförmig gestaltet. Der Rahmen ist gemäß der bevorzugten Ausführungsform mit einem weichelastischen Material 40, ähnlich oder gleich dem Dichtungsmaterial 38, umspritzt, das den Rahmen wie auch Teile seines in Fig. 5 sichtbaren Anschlusses 41 zur Steuerung und Stromversorgung der Verneblungseinrichtung 3 umgibt. Zwischen der radial innen liegenden Umfangsfläche, des die Membran 37 umgebenden Rahmens, bestehend aus dem Rahmen und der Umspritzung 40, und der Membran 37 ist mit Ausnahme der Stege entlang des gesamten Membranumfangs ein Zwischenraum 42 gebildet, der einen Teil eines Umströmungsabschnitts im später erläuterten Strömungskanal des Körpers 1, 2 bildet. Darüber hinaus ist mit Ausnahme des Bereichs des Anschlusses 41 im montierten Zustand ein Abstand zwischen der Außenfläche des Rahmens aus Umspritzung 40 und Rahmen und der Innenumfangsfläche des Körpers (hier ersten Körperteils) ein weiterer Zwischenraum 43 gebildet, der einen weiteren Teil des erwähnten Umströmungskanals bildet. Zur Montage wird die Verneblungseinrichtung 3, die vorgefertigt ist, mit dem Anschluss 41 entsprechend einer Aussparung ausgerichtet, in den ersten Körperteil 1 eingesetzt, wobei die umlaufende Dichtlippe 30 den mit Öffnungen versehenen Teil der Membran 37 umgibt. Im Anschluss wird das zweite Körperteil 2 aufgesetzt, wobei die Vorsprünge 36 gegen den mit elastischem Material 40 umspritzen Rahmen drücken und diesen in Richtung des ersten Körperteils 1 beaufschlagen. Dabei wird die Verneblungseinrichtung 3 in Richtung der Dichtlippe 30 und damit die Membran gegen diese umlaufende Dichtlippe 30 gedrückt, so dass eine Abdichtung gegen die Membran bzw. den die Membran umgebenden Bereich erfolgt und die Fluidkammer 24 dichtend abgeschlossen ist. Der Vernebler wird fertig montiert geliefert und kann auch nicht geöffnet bzw. zerlegt werden.

Darüber hinaus gelangen die konzentrischen Stege 34 und 35 in Eingriff mit dem Vorsprung 29 des Dichtmaterials 28 und bilden die Labyrinthdichtung, wobei der Druck der Dichtungen gegen die entsprechenden Bauteile durch die Verrastung der Verriegelungsnasen 33 durch Hintergreifen des Kragens 27 aufrechterhalten wird. Im Bereich des Anschlusses 41, an dem ein Teil der Vernebelungseinrichtung aus dem Körper 1, 2 tritt, erfolgt eine Abdichtung zwischen dem weichelastischen Kunststoff 40 und den Stegen des zweiten Verriegelungsteils 2 einem eine Aussparung in dem ersten Verriegelungsteil 1 zur Aufnahme des Anschlusses 41 umgebenden Vorsprung 44, so dass auch hier eine ausreichende Abdichtung gegeben ist.

Im montierten Zustand bildet der Körper 1, 2 einen Strömungskanal vom Anschluss 10 über den Anschlussstutzen 11 zum zweiten Anschluss 31 aus dem Anschlussstutzen 32, wobei die Vernebelungseinrichtung 3 entlang der Umströmungskanäle 42, 43 umströmt wird. Die Strömungsrichtung bzw. Umströmung für die Einatemphase ist in den Fig. 6a und b durch die Pfeile dargestellt, während Strömungsrichtung bzw. Umströmung für die Ausatemphase in den Fig. 6c und d durch die Pfeile dargestellt ist. Dabei wird ersichtlich, dass die Strömungsrichtung in den Anschlussstutzen 11 und aus dem Anschlussstutzen 32 gleich gerichtet ist und die Membran 37 bzw. die Ebene, in der die Membran 37 liegt, senkrecht zu dieser Strömungsrichtung bzw. zur Mittelachse der jeweiligen Anschlussstutzen 11, 12 oder 31 ausgerichtet ist. Dadurch erfolgt eine Vernebelung eines in dem Fluidbehälter 14 enthaltenen Fluids durch die Öffnungen der Membran in die Vernebelungskammer 38 bei der dargestellten Ausführungsform in Strömungsrichtung, d. h. parallel dazu. Dadurch wird die Deposition von Fluid an den Oberflächen des Strömungskanals oder in den nachfolgenden Schläuchen vermindert und die Effizienz des Systems erhöht.

Darüber hinaus gestattet diese Ausgestaltung ferner eine Grundströmung von der Zuluftleitung 101 über den Nebenströmungskanal 13 in die Abluftleitung 102 ohne die Vernebelungseinrichtung 3 und insbesondere die Vernebelungskammer 38 zu passieren, so dass diese Grundströmung außerhalb eines Einatemzyklus und/oder Ausatemzyklus kein durch die Vernebelungseinrichtung 3 erzeugtes Aerosol (vernebeltes Fluid) in die Abluftleitung 102 spült, wodurch die Effizienz des Systems weiter verbessert ist (siehe Fig. 6e und f).

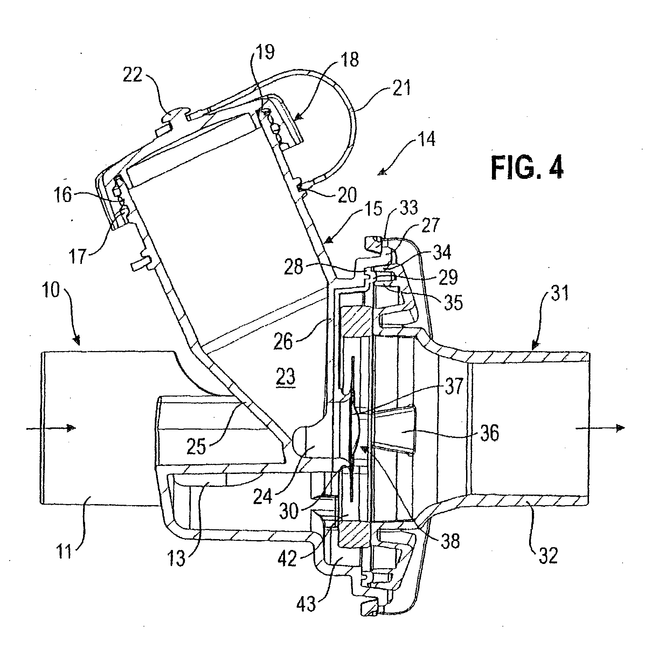

Darüber hinaus wird durch die drei Anschlussstutzen 11, 12 und 32 und die integrale Anbindung des Fluidbehälters 14 an den Körper 1, 2 eine kippstabile Einheit gebildet, die das Ausflussverhalten des, vorzugsweise flüssigen, Fluids aus dem Fluidbehälter 14 in die Fluidkammer 24 und zur Membran 37 begünstigt. Ein gleichmäßiges und konsistentes Zuführen des Fluids wird ferner durch die Ausgestaltung des verjüngten Bereichs 23 und insbesondere die Neigung der Wandung 25 gefördert, so dass selbst bei einer Rotation des in Fig. 4 dargestellten Verneblers um die Mittelachse des Anschlussstutzens 32 um je 45° in eine der beiden Richtungen immer noch ein zuverlässiges Anstehen der Flüssigkeit an der Membran 37 gewährleistet werden kann.

Die Querschnittsfläche des Umströmungskanals 42 und 43 ist dabei derart gestaltet, dass sie nicht wesentlich kleiner und nicht wesentlich größer (letzteres um kein unnötig großes Totraumvolumen zu schaffen, das beim Ausatmen durch den Patienten bei unterstützter Beatmung verdrängt werden muss, zu schaffen) ist als die kleinste Querschnittsfläche in den Leitungen des Beatmungsgeräts zum Patienten 104 (Leitungen 101 und 103). Die Leitungen zum Patienten 103 können sich aus einer sog. Gänsegurgel (Doppeldrehkonnektor) und einem Endotrachealschlauch (Endotrachealtubus) zusammensetzen. Dadurch wird ein erhöhter Strömungswiderstand sowie ein erhöhtes Totraumvolumen verhindert, die beide die Funktionalität des Beatmungsgeräts negativ beeinflussen können.

Darüber hinaus ist durch das Dichtmaterial 28 und die Umspritzung 40 des Rahmens 39 eine Dichtigkeit erreicht, die auch einem Druck von bis zu 100 mbar standhält. Durch die Verwendung der Membran mit den winzigen Öffnungen ist darüber hinaus ein Druckverlust im System bei geöffnetem Fluidbehälter 14 ebenfalls ausgeschlossen. Eine Strömung aus dem Strömungskanal in den Fluidbehälter 14 ist durch die winzigen Öffnungen nicht möglich.

Darüber hinaus kann die Vernebelungseinrichtung 3 über den Anschluss 41 mit der Steuerung des Beatmungsgeräts 100 gekoppelt werden, um eine Triggerung der Vernebelungseinrichtung 3 nur im Einatemzyklus zu bewirken. D. h. nur beim Einatmen des Patienten 104, sei es unterstützt oder zwangsweise durch das Beatmungsgerät 100 vorgegeben, wird die Membran vibriert, so dass eine Vernebelung des Fluids F im Fluidbehälter 14 erfolgt. Dadurch kann die Effizienz noch weiter erhöht werden.

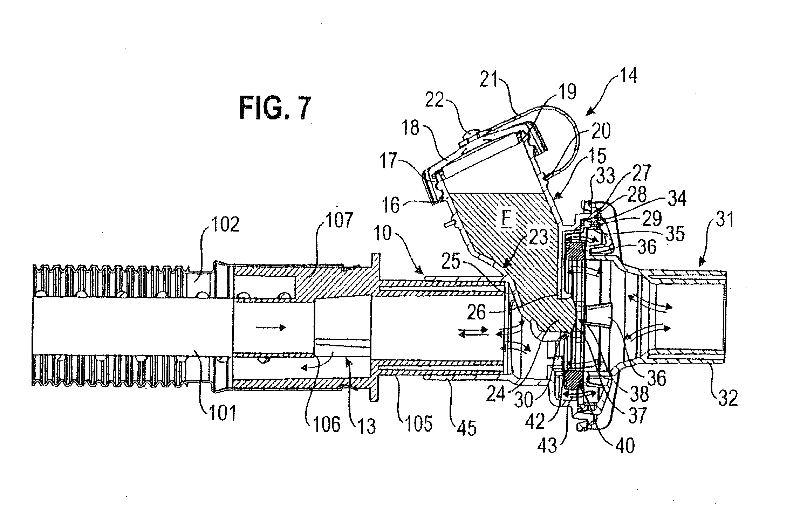

Wie es in Bezug auf die erste Ausführungsform erläutert wurde, sind die Zuluftleitung 101 und die Abluftleitung 102 bei dem dargestellten Vernebler jeweils durch einen separaten Schlauch gebildet, wobei die Schläuche jeweils mit dem Anschlussstutzen 11 bzw. 12 gekoppelt werden. Bei der in Fig. 7 dargestellten zweiten und alternativen Ausführungsform weist der erste Anschluss 10 nur einen Anschlussstutzen 45 auf, in den ein entsprechender Anschlussstutzen 105 einer ersten Leitung 101, 102 des Beatmungsgeräts 100 eingesteckt werden kann. Aus Fig. 7 ist gleichfalls ersichtlich, dass der zweite Anschluss 31 einen Anschlussstutzen 32 aufweist, der im Wesentlichen in seinen Maßen identisch zu dem Anschlussstutzen 105 ist, so dass der Vernebler der vorliegenden Erfindung in bestehende Schlauchsysteme problemlos eingekoppelt werden kann und auch nur in der gedachten Ausrichtung montierbar ist. Hier sind die Leitungen 101 (Zuluft/Abluft), 102 (Abluft/Zuluft) in einem gemeinsamen Schlauch in Form eines Koaxialschlauchs gebildet, wobei die Zuluftleitung 101 durch einen Schlauch mit kleinerem Durchmesser, der in einem Schlauch 102 mit größerem Durchmesser angeordnet und über eine Halterung 107 konzentrisch gehalten ist, ausgestaltet ist. Die Abluftleitung wird durch den Zwischenraum zwischen dem inneren Schlauch und der Außenwand des äußeren Schlauchs 101 gebildet. Die Ausgestaltung kann jedoch auch wie zuvor angedeutet umgekehrt sein. Die Stirnwand 106 des inneren Schlauchs 101, der zum Vernebler gerichtet ist, endet in einem Abstand vor der Membran 37 bzw. der Vernebelungskammer 38, so dass in dem Zwischenraum zwischen der Stirnwand 106 und der Vernebelungseinrichtung 3 der Nebenströmungskanal 13 gebildet wird (durch die Pfeile in Fig. 7 dargestellt) .

Ansonsten unterscheidet sich die Ausführungsform gemäß Fig. 7 nur unwesentlich von der Ausführungsform in den Fig. 1-6, so dass auf weitergehende Ausführungen verzichtet wird und lediglich auf die zuvor beschriebene Ausführungsform verwiesen wird.

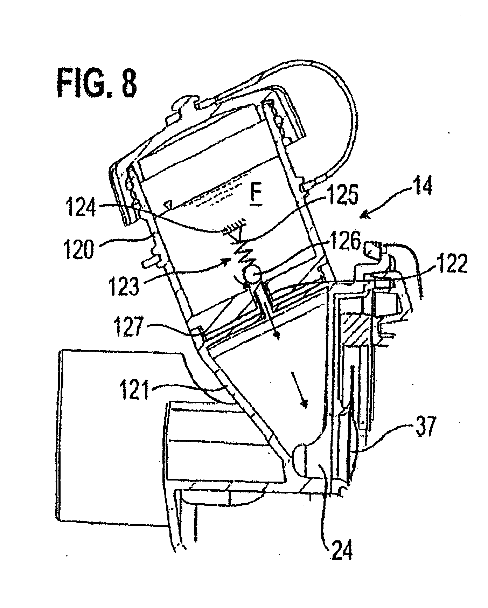

Es versteht sich, dass die vorliegende Erfindung nicht auf die beschriebenen Ausführungsformen beschränkt ist, sondern verschiedenartige Abwandlungen erfolgen können. Z. B. kann anstelle des Koaxialschlauchs aus Fig. 7 auch ein Schlauch mit einer Trennwand vorgesehen werden, um die zwei Leitungen 101, 102 zu bilden. Eine dritte Variante ist ein System von zwei Schläuchen (Zuluft und Abluft), die untrennbar mit einem Y-Stück verbunden sind. Die Einbausituation der Aerosolerzeugungseinrichtung entspricht in diesem Fall auch Fig. 7. Auch ist es denkbar die Membran 37 statt vertikal senkrecht zur Strömungsrichtung in einer gewissen Neigung anzuordnen, die bis zu 45° von der Vertikalen abweichen kann. Wesentlich ist hauptsächlich, dass die Vernebelungseinrichtung in Form der Membran im Strömungsweg liegt und umströmt wird. Erfindungsgemäß ist der Fluidbehälter anders ausgestaltet, wobei der Fluidbehälter selbst über eine Schnittstelle an- und abkoppelbar gestaltet ist. Z. B. könnte der verjüngte Bereich 121 in Fig. 8, der Membran abgewandt abgeschlossen und mit einer Hohlnadel 122 versehen sein, die beim Ankoppeln des zylindrischen Abschnitts 120 ein Ventil 123, das aus einer Kugel 126 und eine die Kugel 126 gegen einen Ventilsitz beaufschlagenden Einrichtung, z.B. einer Feder 125, die sich an einem Festlager 124 in dem zylindrischen Abschnitt 120, der hier auch als Ampulle bezeichnet werden kann, dem Ventilssitz abgewandt abstützt bestehen kann, öffnet, wodurch das Fluid auf einmal oder allmählich in die Fluidkammer 24 bzw. den verjüngten Teil 121 strömen kann. Dabei kann das Ventil z. B. direkt beim Aufsetzen des zylindrischen Teils 120 auf den verjüngten Teil 121 z.B. über ein Schraubgewinde 127, sobald eine im Wesentlichen dichtende Verbindung zwischen diesen Elementen vorliegt, automatisch geöffnet werden (siehe schematisch in Fig. 8).

Darüber hinaus ist prinzipiell auch die Verwendung einer anderen Vernebelungseinrichtung als der dargestellten denkbar, so könnten z. B. auch Düsenvernebler zum Einsatz kommen, insofern die Vernebelungsrichtung im Wesentlichen in, d. h. parallel zur Strömungsrichtung zwischen den Anschlüssen 10 und 31 liegt. Darüber hinaus ist es auch denkbar den Vernebler in der Leitung 101 anzuordnen und die Verbindung der Leitungen 101, 102 und 103 über ein Y-Stück zu gestalten. Dann ist jedoch die Effizienz vermindert, da die Grundströmung stets Aerosol in die Abluftleitung 102 transportieren würde, es sei denn es wird eine Triggerung der Vernebelungseinrichtung 3 in Entsprechung mit dem Einatemzyklus vorgesehen, so dass während der Grundströmung, ohne dass ein Einatmen stattfindet, keine Vernebelung erfolgt. Auch wäre es im Prinzip denkbar den Vernebler nach einem Y-Stück anzuordnen, d. h. der Vernebler, wie er in Fig. 7 dargestellt ist, könnte unmittelbar an ein Y-Stück angeschlossen werden, d. h. zwischen Y-Stück und der Leitung 103 (Gänsegurgel und Endotrachaeltubus) sowie den mit dem Y-Stück gekoppelten Leitungen 101, 102. Jedoch ist insbesondere die Integration von Y-Stück und Vernebler, wie sie in der ersten Ausführungsform beschrieben ist, besonders bevorzugt.

Schließlich können folgende jedoch nicht abschließend aufgelistete Wirkstoffklassen bzw. -substanzen durch den Vernebler der vorliegenden Erfindung vernebelt werden:
Unter den wirksamen Verbindungen sind beispielsweise Substanzen, die aus der Gruppe ausgewählt sind, die aus antientzündlichen Verbindungen, Glucokorticoiden, Medikamenten gegen Allergien, Antioxidantien, Vitaminen, Leukotrien-Antagonisten, Mitteln gegen Infektionen, Antibiotika, Antifungiziden, Antivirusmitteln, Mucolytica, Dekongestiva, Antiseptika, Cytostatika, Immunmodulatoren, Impfstoffen, Mitteln zur Wundheilung, Lokalanästhetika, Oligonukleotiden, Peptiden, Proteinen und Pflanzenextrakten besteht.

Beispiele für möglicherweise nützliche anti-entzündliche Verbindungen sind Glucokorticoide und nicht-steroidale anti-entzündliche Mittel, wie beispielsweise Betamethason, Beclomethason, Budesonid, Ciclesonid, Dexamethason, Desoxymethason, Fluoconolonacetonid, Flucinonid, Flunisolid, Fluticason, Icomethason, Rofleponid, Triamcinolonacetonid, Fluorcortinbutyl, Hydrocortison, Hydroxycortison-17-Butyrat, Prednicarbat, 6-Methylprednisolonaceponat, Mometasonfuroat, Dehydroepiandrosteronsulfat (DHEAS), Elastan, Prostaglandin, Leukotrin, Bradykinin-Antagonisten, nicht-steroidale anti-entzündliche Medikamente (NSAIDs), wie beispielsweise Ibuprofen, einschließlich jeglicher pharmazeutisch annehmbarer Salze, Ester, Isomere, Stereoisomere, Diastereomere, Epimere, Solvate oder andere Hydrate davon, Promedikamente, Derivate oder irgendwelche anderen chemischen oder physikalischen Formen wirksamer Verbindungen, die die entsprechenden wirksamen Reste umfassen.

Beispiele für Mittel gegen Infektionen, deren Klasse oder therapeutische Kategorie hier als solche Verbindungen umfassend verstanden wird, die gegen bakterielle, pilzliche und virale Infektionen wirksam sind, d. h. solche, die die Klassen der Mikrobizide, Antibiotika, Fungizide, Antiseptika und Anti-Virus-Mittel umfassen, sind
- Penizilline, einschließlich Benzylpenizilline (Penizillin-G-Natrium, Clemizon-Penizillin, Benzathin-Penizillin G), Phenoxypenizilline (Penizillin V, Propizillin), Aminobenzylpenizilline (Ampizillin, Amoxyzillin, Bacampizillin), Acylaminopenizilline (Azlozillin, Mezlozillin, Piperazillin, Apalzillin), Carboxypenizilline (Carbenizillin, Ticarzillin, Temozillin), Isoxazolyl-Penizilline (Oxazillin, Cloxazillin, Dicloxazillin, Flucloxazillin) und Amiidin-Penizilline (Mecillinam);
- Cephalosporine, einschließlich Cefazoline (Cefazolin, Cefazedon);Cefuroxime (Cerufoxim, Cefamdol, Cefotiam), Cefoxitine (Cefoxitin, Cefotetan, Latamoxef, Flomoxef), Cefotaxime (Cefotaxim, Ceftriaxon, Ceftizoxim, Cefmenoxim), Ceftazidime (Ceftazidim, Cefpirom, Cefepim), Cefalexine (Cefalexin, Cefaclor, Cefadroxil, Cefradin, Loracarbef, Cefprozil) und Cefixime (Cefixim, Cefpodoxim-Proxetil, Cefuroxime-Axetil, Cefetamet-Pivoxil, Cefotiam-Hexetil), Loracarbef, Cefepim, Clavulansäure/Amoxizillin, Ceftobiprol;
- Synergisten, einschließlich Beta-Lactamase-Inhibitoren, wie beispielsweise Clavulansäure, Sulbactam und Tazobactam;
- Carbapeneme, einschließlich Imipenem, Cilastin, Meropenem, Doripenem, Tebipenem, Ertapenem, Ritipenam und Biapenem;
- Monobactame, einschließlich Aztreonam;
- Aminoglycoside, wie beispielsweise Apramycin, Gentamycin, Amikacin, Isepamicin, Arbekacin, Tobramycin, Netilmicin, Spectinomycin, Streptomycin, Capreomycin, Neomycin, Paromoycin und Kanamycin;
- Macrolide, einschließlich Erythromycin, Clarythromycin, Roxithromycin, Azithromycin, Dithromycin, Josamycin, Spiramycin und Telithromycin;
- Gyrase-Inhibitoren oder Flurochinolone, einschließlich Ciprofloxacin, Gatifloxacin, Norfloacin, Ofloxycin, Levofloxacin, Perfloxacin, Lomefloxacin, Garenoxacin, Clinafloxacin, Sitafloxacin, Prulifloxacin, Olamufloxacin, Caderofloxacin, Gemifloxacin, Balofloxacin, Trovafloxacin und Moxifloxacin;
- Tetracycline, einschließlich Tetracyclin, Oxytetracyclin, Rolitetracyclin, Minocyclin, Doxycyclin, Tigecyclin und Aminocyclin;
- Glycopeptide, einschließlich Vacomycin, Teicoplanin, Ristocetin, Avoparcin, Oritavancin, Ramoplanin und Peptide 4;
- Polypeptide, einschließlich Plectasin, Dalbavancin, Daptomycin, Oritavancin, Ramoplanin, Dalbavancin, Telavancin, Bacitracin, Tyrothricin, Neomycin, Kanamycin, Mupirocin, Paromomycin, Polymyxin B und Colistin;
- Sulfonamide, einschließlich Sulfadiazin, Sulfamethoxazol, Sulfalen, Co-Trimoxazol, Co-Trimetrol, Co-Trimoxazin und Co-Tetraxazin;
- Azole, einschließlich Clotrimazol, Oxiconazol, Miconazol, Ketoconazol, Itraconazol, Fluconazol, Metronidazol, Tinidazol, Bifonazol, Ravuconazol, Posaconazol, Voriconazol und Ornidazol und andere Antifungizide, einschließlich Flucytosin, Griseofluvin, Tonoftal, Naftifin, Terbinafin, Amorolfin, Ciclopiroxolamin, Echinocandin, wie Micafungin, Caspofungin, Anidulafungin;
- Nitrofurane, einschließlich Nitrofurantoin und Nitrofuranzon;
- Polyene, einschließlich Amphotericin B, Natamycin, Nystatin, Flucocytosin;
- andere Antibiotika, einschließlich Tithromycin, Lincomycin, clindamycin, Oxazolindione (Linzezolide), Ranbezolid, Streptogramin A+B, Pristinamycin aA+B, Virginiamycin A+B, Dalfopristin/Chiunupristin (Synercid), Chloramphenicol, Ethambutol, Pyrazinamid, Terizidon, Dapson, Prothionamid, Fosfomycin, Fucidinsäure, Rifampicin, Isoniazid, Cycloserin, Terizidon, Ansamycin, Lysostaphin, Iclaprim, Mirocin B17, Clerocidin, Filgrastim und Pentamidin;
- Antivirusmittel, einschließlich Aziclovir, Ganciclovir, Birivudin, Valaciclovir, Zidovudin, Didanosin, Thiacytidin, Stavudin, Lamivudin, Zalcitabin, Ribavirin, Nevirapirin, Delaviridin, Trifulridin, Ritonavir, Saquinavir, Indinavir, Foscarnet, Amantadin, Podophyllotoxin, Vidarabin, Tromantadin und Proteinase-Inhibitoren;
- Antiseptica, einschließlich Acridin-Derivate, Iod-Povidon, Benzoate, Rivanol, Chlorhexidin, quarternäre Ammonium-Verbindungen, Cetrimide, Biphenylol, Clorofen und Octenidin;
- Pflanzenextrakte oder Inhaltsstoffe, wie beispielsweise Pflanzenextrakte aus Kamille, Hamamelis, Echinacea, Calendula, Thymian, Papain, Pelagonien, Kiefernbäumen, ätherische Öle, Myrtol, Pinen, Limonen, Cineol, Thymol, Menthol, Kampfer, Tannin, Alpha-Hederin, Bisabolol, Lycopodin, Vitapherol;
- Verbindungen zur Wundheilung einschließlich Dexpantenol, Allantoin, Vitamine, Hyaluronsäure, Alpha 1-Antitrypsin, anorganische und organische Zinksalze/-verbindungen, Bismuth- und Selen-Salze;
- Interferone (Alpha, Beta, Gamma), Tumor-Nekrose-Faktoren, Cytokine, Interleukine;
- Immunmodulatoren einschließlich Methotrexat, Azathioprin, Cyclosporin, Tacrolismus, Sirolimus, Rapamycin, Mofetil; Mofetil-mycophenolat.
- Cytostatika und Metastase-Inhibitoren;
- Alkyliermittel, wie Nimustin, Melphanalan, Carmustin, Lomustin, Cyclophosphosphamid, Ifosfamid, Trofosamid, Chlorambucil, Busulfan, Treosulfan, Prednimustin, Thiotepa;
- Antimetabolite, z. B. Cytarabin, Fluoruracil, Methotrexat, Mercaptopurin, Thioguanin;
- Alkaloide, wie Vinblastin, Vincristin, Vindesin;
- Antibiotika, wie beispielsweise Alcarubicin, Bleomycin, Dactinomycin, Daunorubicin, Doxorubicin, Epirubicin, Idarubicin, Mitomycin, Plicamycin;
- Komplexe von Elementen der Übergangsgruppen (z. B. Ti, Zr, V, Nb, Ta, Mo, W, Pt) wie beispielsweise Carboplatin, Cis-Platin und Metallocenverbindungen, wie beispielsweise Titanocendichlorid;
- Amsacrin, Dacarbazin, Estramustin, Etoposid, Beraprost, Hydroxycarbamid, Mitoxanthron, Procarbazin, Temiposid;
- Paclitaxel, Iressa, Zactima, Poly-ADP-Ribose-Polymerase (PRAP)-Enzyminhibitoren, Banoxantron, Gemcitabin, Pemetrexed, Bevacizumab, Ranibizumab.

Beispiele möglicherweise nützlicher Mucolide sind DNase, P2Y2-Agonisten (Denufosol), Medikamente, die das Eindringen von Chlor- und Natrium betreffen, wie beispielsweise N-(3,5-diamino-6-chlorpyrazin-2-carbony)-N'-{4-[4-(2,3-dihydroxypropoxy) -phenyl]butyl}guanidin-Methanesulfonat (PARION 552-02) Heparinoide, Guaifenesin, Acetylcystein, Carbocystein, Ambroxol, Bromhexin, Tyloxapol, Lecithine, Myrtol und rekombinante Tensid-Proteine.

Beispiele potenziell nützlicher Vasokonstriktoren und Dekongestiva, die nützlich sein können, um die Schwellung der Schleimhaut zu reduzieren, sind Phenylephrin, Naphazolin, Tramazolin, Tetryzolin, Oxymetazolin, Fenoxazolin, Xylometazolin, Epinephrin, Isoprenalin, Hexoprenalin und Ephedrin.

Beispiele für potenziell nützliche Lokalanästhetika schließen Bezocain, Tetracain, Procain, Lidocain und Bupivacain ein.

Beispiele möglicher nützlicher Mittel gegen Allergien schließen die oben erwähnten Glucocorticoide, Cromolyn-Natrium, Nedocromil, Cetrizin, Loratidin, Montelukast, Roflumilast, Ziluton, Omalizumab, Heparinoide und andere Anthistaminika, einschließlich Azelastin, Cetirizin, Desloratadin, Ebastin, Fexofenadin, Levocetirizin, Loratadin ein.

Antisense-Oligonucleotide sind kurze synthetische Stränge aus DNA (oder Analoga), die komplementär oder im Gegensinn zur Zielsequenz (DNA, RNA) sind, welche so entworfen sind, dass sie einen biologischen Vorgang stoppen, wie beispielsweise die Transkription, Translation oder das Splicing. Die dadurch hervorgerufene Inhibierung der Genexpression macht Oligonucleotide, abhängig von ihrer Zusammensetzung, nützlich für die Behandlung vieler Erkrankungen, und zahlreiche Verbindungen werden derzeit klinisch untersucht, wie beispielsweise ALN-RSV01, um das respiratorische Syncytiumvirus zu behandeln, AVE-7279, um Asthma und Allergien zu behandeln, TPI-ASM8, um allergisches Asthma zu behandeln, 1018-ISS, um Krebs zu behandeln.

Beispiele potenziell nützlicher Peptide und Proteine schließen Aminosäuren ein, wie beispielsweise L-Arginin, L-Lysin, Antikörper gegen Toxine, die von Mikroorganismen hergestellt werden, antimikrobielle Peptide, wie beispielsweise Cecropine, Defensine, Thionine und Cathelicidine.

Für jedes dieser und anderer explizit erwähnten Beispiele für Medikamentensubstanzen, die potenziell zur Durchführung der Erfindung nützlich sind, sollen die Verbindungsnamen, die hier angegeben worden sind, so verstanden werden, dass sie auch jegliche pharmazeutisch annehmbaren Salze, Solvate oder andere Hydrate, Promedikamente, Isomere oder irgendwelche anderen chemischen oder physikalischen Formen der entsprechenden Verbindungen, die die entsprechenden aktiven Reste umfassen, betreffen.
1. Vernebler für Beatmungsmaschinen, umfassend
   einen Körper (1, 2) mit einem ersten Anschluss (10) zur Verbindung des Verneblers mit einem Beatmungsgerät (100) und einem zweiten Anschluss (31) zur Verbindung des Verneblers mit einer zu einem Patienten führenden Leitung (103), wobei der Körper einen Strömungskanal von dem ersten Anschluss (10) zu dem zweiten Anschluss (31) bildet; und
   eine Verneblungseinrichtung (3) zur Vernebelung eines Fluids;
   **dadurch gekennzeichnet, dass** die Verneblungseinrichtung (3) derart ausgestaltet und zwischen dem ersten Anschluss (10) und dem zweiten Anschluss (31) in dem Strömungskanal angeordnet ist, dass das Fluid im Wesentlichen parallel zur, vorzugsweise in, Strömungsrichtung vom ersten Anschluss zum zweiten Anschluss vernebelbar ist.
2. Vernebler nach 1, bei dem der erste Anschluss (10) derart zur Verbindung mit einer Zuluftleitung (101) von dem Beatmungsgerät (100) und einer Abluftleitung (102) zu dem Beatmungsgerät (100) gestaltet ist, dass auf der dem zweiten Anschluss (31) entgegengesetzten Seite der Vernebelungseinrichtung (3) ein Nebenströmungskanal (13) von der Zuluftleitung (101) in die Abluftleitung (102) gebildet ist.
3. Vernebler nach 2, bei dem der erste Anschluss (10) einen ersten Anschlussstutzen (11) zur Verbindung mit der Zuluftleitung (101) und einen zweiten Anschlussstutzen (12) zur Verbindung mit der Abluftleitung (102) aufweist.
4. Vernebler nach einem der vorstehenden, bei dem die Vernebelungseinrichtung (3) eine vibrierbare Membran (37) umfasst, die zur Vernebelung des Fluids mit einer Vielzahl von Öffnungen versehen und im Wesentlichen senkrecht zur Strömungsrichtung vom ersten Anschluss (10) zum zweiten Anschluss (31) angeordnet ist.
5. Vernebler nach 4, ferner umfassend einen mit dem Körper (1, 2) verbundenen Fluidbehälter (14) zur Aufnahme des zu vernebelnden Fluids; und
   eine Verneblungskammer (38) in die das Fluid zu vernebeln ist, wobei die Membran (37) zwischen dem Fluidbehälter (14) und der Verneblungskammer (38) angeordnet ist.
6. Vernebler nach 5, bei dem die Vernebelungskammer (38) zwischen der Membran (14) und dem zweiten Anschluss (31) liegt.
7. Vernebler nach 5 oder 6, bei dem der Fluidbehälter (14) eine Verjüngung (23) zur Membran (14) hin umfasst, die in eine durch die Membran abgeschlossene Fluidkammer (24) mündet.
8. Vernebler nach 7, bei dem sich zumindest ein der Membran (37) abgewandter Teilbereich (25) der Verjüngung (23) in einem Winkelbereich zwischen 10° und 40° zur Vertikalen erstreckt.
9. Vernebler nach einem der 5 bis 8, bei dem der Strömungskanal einen Umströmungsabschnitt (42, 43) aufweist, der in Radialrichtung zwischen der Membran (37) und dem Körper (1, 2) ausgebildet ist und in etwa eine Querschnittsfläche entsprechend der kleinsten Querschnittsfläche einer Leitung des Beatmungsgeräts (100) zum Patienten aufweist.
10. Vernebler nach 9, bei dem die Membran (37) über Stege in einem die Membran (37) umgebenden Rahmen (19, 40) aufgehängt ist, so dass zwischen dem Rahmen (19, 40) und der Membran (37) zumindest ein Teil (42) des Umströmungsabschnitts (42, 43) gebildet ist.
11. Vernebler nach einem der 5 bis 9, bei dem in dem Fluidbehälter eine Fluidkommunikationsschnittstelle vorgesehen ist und der Fluidbehälter zur Aufnahme einer durch die Fluidkommunikationsschnittstelle in Fluidverbindung mit dem Fluidbehälter gelangenden Fluid enthaltenden Ampulle ausgestaltet ist.
12. Beatmungsmaschine, umfassend ein Beatmungsgerät mit einer Zuluftleitung (101), um Atemluft zur Verfügung zu stellen, und einer Abluftleitung (102), um verbrauchte Luft zurückzuführen, und eine zweite Leitung (103), die zu einem Patienten (104) führt, sowie einen Vernebler nach einem der vorstehenden, wobei der erste Anschluss (10) des Körpers (1, 2) zumindest mit der Zufluftleitung (101) verbunden ist.
13. Beatmungsmaschine nach 12, bei der der erste Anschluss (10) des Körpers (1, 2) ferner mit der Abluftleitung (102) verbunden ist und der zweite Anschluss (31) des Körpers mit der zweiten Leitung (103) verbunden ist.
14. Beatmungsmaschine nach 13, bei der die Zuluftleitung (101) und die Abluftleitung (102) jeweils durch einen separaten Schlauch gebildet sind, die mit dem entsprechenden ersten (11) und zweiten (12) Anschlussstutzen des ersten Anschlusses (10) eines Verneblers mit den Merkmalen des Anspruchs 3 gekoppelt sind und das Beatmungsgerät (100) derart gestaltet ist, dass eine kontinuierliche Grundströmung erzeugbar ist, die während des Einatemzyklus zusammen mit Beatmungsluft von der Zuluftleitung (101) durch den Strömungskanal des Körpers (1, 2) in die zweiten Leitung (103) zum Patienten (104) strömt und sonst über die Zuluftleitung (101) und den Nebenströmungskanal (13) eines Verneblers mit den Merkmalen des Anspruchs 2 in die Abluftleitung (102) strömbar ist.
15. Beatmungsmaschine nach 13, bei der die Zuluftleitung (101) und die Abluftleitung (102) durch einen gemeinsamen durch eine Trennwand oder in Form eines Koaxialschlauchs in zwei Abschnitte unterteilten Schlauch gebildet sind, der mit dem ersten Anschluss (10) des Verneblers gekoppelt ist und das Beatmungsgerät (100) derart gestaltet ist, dass eine kontinuierliche Grundströmung erzeugbar ist, die während des Einatemzyklus zusammen mit Beatmungsluft von der Zuluftleitung (101) durch den Strömungskanal des Körpers (1, 2) in die zweite Leitung (103) zum Patienten (104) strömt und sonst über die Zuluftleitung (101) und den Nebenströmungskanal (13) eines Verneblers mit den Merkmalen des Anspruchs 2, der zwischen der der Verneblungseinrichtung zugewandten Stirnseite der Trennwand oder des inneren Schlauchs des Koaxialschlauchs und der Verneblungseinrichtung gebildet ist, in die Abluftleitung (103) strömbar ist.
16. Beatmungsmaschine nach einem der Ansprüche 12 bis 15, ferner umfassend eine Steuerung über die die Vernebelungseinrichtung (3) entsprechend dem Einatemzyklus triggerbar ist, so dass eine Vernebelung des Fluids nur während des Einatemzyklus auslösbar ist.

## Patentansprüche

1. Vernebler für Beatmungsmaschinen, umfassend
einen Körper (1, 2) mit einem ersten Anschluss (10) zur Verbindung des Verneblers mit einem Beatmungsgerät (100) und einem zweiten Anschluss (31) zur Verbindung des Verneblers mit einer zu einem Patienten führenden Leitung (103), wobei der Körper einen Strömungskanal von dem ersten Anschluss (10) zu dem zweiten Anschluss (31) bildet;
eine Verneblungseinrichtung (3) zur Vernebelung eines Fluids, die derart ausgestaltet und zwischen dem ersten Anschluss (10) und dem zweiten Anschluss (31) in dem Strömungskanal angeordnet ist, dass sie im Einatemzyklus von Beatmungsluft umströmbar ist und Fluid im Wesentlichen parallel zur, vorzugsweise in, Strömungsrichtung vom ersten Anschluss zum zweiten Anschluss vernebelbar ist, wobei die Vernebelungseinrichtung (3) eine vibrierbare Membran (37) umfasst, die zur Vernebelung des Fluids mit einer Vielzahl von Öffnungen versehen ist;
einen mit dem Körper (1, 2) verbundenen Fluidbehälter (14) zur Aufnahme des zu vernebelnden Fluids; und
eine Verneblungskammer (38), in die das Fluid zu vernebeln ist, wobei die Membran (37) zwischen dem Fluidbehälter (14) und der Verneblungskammer (38) angeordnet ist, **dadurch gekennzeichnet, dass** der Fluidbehälter (14) an- und abkoppelbar mit dem Körper verbunden ist

2. Vernebler nach Anspruch 1, bei dem die Vernebelungskammer (38) zwischen der Membran (14) und dem zweiten Anschluss (31) liegt.

3. Vernebler nach Anspruch 1 oder 2, bei dem der Fluidbehälter (14) eine Verjüngung (23) zur Membran (14) hin umfasst, die in eine durch die Membran abgeschlossene Fluidkammer (24) mündet.

4. Vernebler nach Anspruch 3, bei dem sich zumindest ein der Membran (37) abgewandter Teilbereich (25) der Verjüngung (23) in einem Winkelbereich zwischen 10° und 40° zur Vertikalen erstreckt.

5. Vernebler nach einem der Ansprüche 1 bis 4, bei dem der Strömungskanal einen Umströmungsabschnitt (42, 43) aufweist, der in Radialrichtung zwischen der Membran (37) und dem Körper (1, 2) ausgebildet ist und in etwa eine Querschnittsfläche entsprechend der kleinsten Querschnittsfläche einer Leitung des Beatmungsgeräts (100) zum Patienten aufweist.

6. Vernebler nach Anspruch 5, bei dem die Membran (37) über Stege in einem die Membran (37) umgebenden Rahmen (19, 40) aufgehängt ist, so dass zwischen dem Rahmen (19, 40) und der Membran (37) zumindest ein Teil (42) des Umströmungsabschnitts (42, 43) gebildet ist.

7. Vernebler nach einem der vorstehenden Ansprüche, bei dem die Membran (37) im Wesentlichen senkrecht zur Strömungsrichtung vom ersten Anschluss (10) zum zweiten Anschluss (31) angeordnet ist.

8. Beatmungsmaschine, umfassend ein Beatmungsgerät mit einer Zuluftleitung (101), um Atemluft zur Verfügung zu stellen, und einer Abluftleitung (102), um verbrauchte Luft zurückzuführen, und eine zweite Leitung (103), die zu einem Patienten (104) führt, sowie einen Vernebler nach einem der vorstehenden Ansprüche, wobei der erste Anschluss (10) des Körpers (1, 2) zumindest mit der Zuluftleitung (101) verbunden ist.

9. Beatmungsmaschine nach Anspruch 8, bei der der erste Anschluss (10) des Körpers (1, 2) ferner mit der Abluftleitung (102) verbunden ist und der zweite Anschluss (31) des Körpers mit der zweiten Leitung (103) verbunden ist.

10. Beatmungsmaschine nach Anspruch 9, bei der die Zuluftleitung (101) und die Abluftleitung (102) jeweils durch einen separaten Schlauch gebildet sind, die mit dem entsprechenden ersten (11) und zweiten (12) Anschlussstutzen des ersten Anschlusses (10) eines Verneblers mit den Merkmalen des Anspruchs 3 gekoppelt sind und das Beatmungsgerät (100) derart gestaltet ist, dass eine kontinuierliche Grundströmung erzeugbar ist, die während des Einatemzyklus zusammen mit Beatmungsluft von der Zuluftleitung (101) durch den Strömungskanal des Körpers (1, 2) in die zweiten Leitung (103) zum Patienten (104) strömt und sonst über die Zuluftleitung (101) und den Nebenströmungskanal (13) eines Verneblers mit den Merkmalen des Anspruchs 2 in die Abluftleitung (102) strömbar ist.

11. Beatmungsmaschine nach Anspruch 9, bei der die Zuluftleitung (101) und die Abluftleitung (102) durch einen gemeinsamen durch eine Trennwand oder in Form eines Koaxialschlauchs in zwei Abschnitte unterteilten Schlauch gebildet sind, der mit dem ersten Anschluss (10) des Verneblers gekoppelt ist und das Beatmungsgerät (100) derart gestaltet ist, dass eine kontinuierliche Grundströmung erzeugbar ist, die während des Einatemzyklus zusammen mit Beatmungsluft von der Zuluftleitung (101) durch den Strömungskanal des Körpers (1, 2) in die zweite Leitung (103) zum Patienten (104) strömt und sonst über die Zuluftleitung (101) und den Nebenströmungskanal (13) eines Verneblers mit den Merkmalen des Anspruchs 2, der zwischen der der Verneblungseinrichtung zugewandten Stirnseite der Trennwand oder des inneren Schlauchs des Koaxialschlauchs und der Verneblungseinrichtung gebildet ist, in die Abluftleitung (103) strömbar ist.

12. Beatmungsmaschine nach einem der Ansprüche 8 bis 11, ferner umfassend eine Steuerung über die die Vernebelungseinrichtung (3) entsprechend dem Einatemzyklus triggerbar ist, so dass eine Vernebelung des Fluids nur während des Einatemzyklus auslösbar ist.

## Claims

1. Nebuliser for artificial respiration machines comprising
a body (1, 2) with a first connection (10) for connecting the nebuliser to an artificial respiration device (100) and a second connection (31) for connecting the nebuliser to a pipe (103) leading to a patient, wherein the body forms a flow channel from the first connection (10) to the second connection (31);
a nebulising device (3) for nebulising a fluid which is designed and arranged in the flow channel between the first connection (10) and the second connection (31) such that it can be flowed around in the inhalation cycle by artificial respiration air and fluid can be nebulised essentially parallel to, preferably in, the flow direction from the first connection to the second connection, wherein the nebulising device (3) comprises a vibratable membrane (37) which is provided for nebulising the fluid using a plurality of openings;
a fluid container (14) connected to the body (1, 2) to receive the fluid to be nebulised; and
a nebulising chamber (38) into which the fluid is to be nebulised, wherein the membrane (37) is arranged between the fluid container (14) and the nebulising chamber (38), **characterised in that** the fluid container (14) is connected to the body to be able to be coupled and uncoupled.

2. Nebuliser according to claim 1, in which the nebulising chamber (38) lies between the membrane (14) and the second connection (31).

3. Nebuliser according to claim 1 or 2, in which the fluid container (14) comprises tapering (23) towards the membrane (14) which leads into a fluid chamber (24) closed by the membrane.

4. Nebuliser according to claim 3, in which at least one part region (25) of the tapering (23) facing away from the membrane (37) extends at an angle range between 10° and 40° to the vertical.

5. Nebuliser according to one of claims 1 to 4, in which the flow channel has a flow-around section (42, 43) which is formed in the radial direction between the membrane (37) and the body (1, 2) and has approximately a cross-sectional area corresponding to the smallest cross-sectional area of a pipe of the artificial respiration device (100) to the patient.

6. Nebuliser according to claim 5, in which the membrane (37) is suspended via bars in a frame (19, 40) surrounding the membrane (37) so that at least one part (42) of the flow-around section (42, 43) is formed between the frame (19, 40) and the membrane (37).

7. Nebuliser according to one of the preceding claims, in which the membrane (37) is arranged essentially vertically to the flow direction from the first connection (10) to the second connection (31).

8. Artificial respiration machine comprising an artificial respiration device with an incoming air pipe (101) to make available respiratory air, and an outgoing air pipe (102) to return spent air, and a second pipe (103) which leads to a patient (104), and a nebuliser according to one of the preceding claims, wherein the first connection (10) of the body (1, 2) is connected at least to the ingoing air pipe (101).

9. Artificial respiration machine according to claim 8, in which the first connection (10) of the body (1, 2) is connected further to the outgoing air pipe (102) and the second connection (31) of the body is connected to the second pipe (103).

10. Artificial respiration machine according to claim 9, in which the ingoing air pipe (101) and the outgoing air pipe (102) are formed in each case by a separate tube which are coupled to the corresponding first (11) and second (12) connection pieces of the first connection (10) of a nebuliser having the features of claim 3 and the artificial respiration device (100) is designed such that a continuous residual flow can be generated which during the inhalation cycle flows together with artificial respiration air from the ingoing air pipe (101) through the flow channel of the body (1, 2) into the second pipe (103) to the patient (104) and otherwise can flow via the ingoing air pipe (101) and the subsidiary flow channel (13) of a nebuliser having the features of claim 2 into the outgoing air pipe (102).

11. Artificial respiration machine according to claim 9, in which the ingoing air pipe (101) and the outgoing air pipe (102) are formed by a common tube divided into two sections by a separating wall or in the form of a coaxial tube and which is coupled to the first connection (10) of the nebuliser and the artificial respiration device (100) is designed such that a continuous residual flow can be generated which during the inhalation cycle flows together with artificial respiration air from the ingoing air pipe (101) through the flow channel of the body (1, 2) into the second pipe (103) to the patient (104) and otherwise can flow via the ingoing air pipe (101) and the subsidiary flow channel (13) of a nebuliser having the features of claim 2 which is formed between the end-face side facing the nebulising device of the separating wall or of the inner tube of the coaxial tube and of the nebulising device into the outgoing air pipe (103).

12. Artificial respiration machine according to one of claims 8 to 11, further comprising a control by means of which the nebulising device (3) can be triggered according to the inhalation cycle so that nebulisation of the fluid can be triggered only during the inhalation cycle.

## Revendications

1. Nébuliseur pour des dispositifs d'assistance respiratoire, comportant
un corps (1, 2) avec un premier raccord (10) pour relier le nébuliseur à un appareil d'assistance respiratoire (100) et un deuxième raccord (31) pour relier le nébuliseur à une conduite (103) menant vers un patient, ledit corps formant un canal de circulation depuis le premier raccord (10) vers le deuxième raccord (31) ;
un dispositif de nébulisation (3) destiné à nébuliser un fluide, lequel dispositif est configuré et est disposé entre le premier raccord (10) et le deuxième raccord (31) dans le canal de circulation, de telle sorte que pendant le cycle d'inspiration l'air de ventilation contourne ledit dispositif de nébulisation et le fluide peut être nébulisé sensiblement parallèlement à, de préférence dans la direction de circulation depuis le premier raccord vers le deuxième raccord, le dispositif de nébulisation (3) comportant une membrane (37) susceptible de vibrer, qui est munie d'une pluralité d'ouvertures pour la nébulisation du fluide ;
un réservoir de fluide (14), relié au corps (1, 2) et destiné à recevoir le fluide à nébuliser ; et
une chambre de nébulisation (38), dans laquelle le fluide doit être nébulisé, ladite membrane (37) étant disposée entre le réservoir de fluide (14) et la chambre de nébulisation (38), **caractérisé en ce que** le réservoir de fluide (14) est relié au corps de manière à pouvoir être couplé ou découplé de celui-ci.

2. Nébuliseur selon la revendication 1, dans lequel la chambre de nébulisation (38) se situe entre la membrane (14) et le deuxième raccord (31).

3. Nébuliseur selon la revendication 1 ou 2, dans lequel le réservoir de fluide (14) comporte un rétrécissement (23), qui est orienté vers la membrane (14) et débouche dans une chambre de fluide (24) fermée par la membrane.

4. Nébuliseur selon la revendication 3, dans lequel au moins une zone partielle (25) du rétrécissement (23), opposée à la membrane (37), s'étend dans une zone angulaire entre 10° et 40° par rapport à la verticale.

5. Nébuliseur selon l'une quelconque des revendications 1 à 4, dans lequel le canal de circulation comporte un tronçon de contournement (42, 43) qui est réalisé dans la direction radiale entre la membrane (37) et le corps (1, 2) et comporte une section transversale correspondant à peu près à la plus petite section transversale d'une conduite de l'appareil d'assistance respiratoire (100) vers le patient.

6. Nébuliseur selon la revendication 5, dans lequel la membrane (37) est suspendue par des barrettes dans un cadre (19, 40) entourant la membrane (37), de telle sorte qu'au moins une partie (42) du tronçon de contournement (42, 43) est formée entre le cadre (19, 40) et la membrane (37).

7. Nébuliseur selon l'une quelconque des revendications précédentes, dans lequel la membrane (37) est disposée sensiblement perpendiculairement à la direction de circulation depuis le premier raccord (10) vers le deuxième raccord (31).

8. Dispositif d'assistance respiratoire, comportant un appareil d'assistance respiratoire avec une conduite d'air frais (101), permettant d'amener de l'air à inhaler, et une conduite d'air vicié (102) permettant de renvoyer l'air usagé, et une deuxième conduite (103) qui mène vers un patient (104), ainsi qu'un nébuliseur selon l'une quelconque des revendications précédentes, le premier raccord (10) du corps (1, 2) étant relié au moins à la conduite d'air frais (101).

9. Dispositif d'assistance respiratoire selon la revendication 8, dans lequel le premier raccord (10) du corps (1, 2) est relié, en outre, à la conduite d'air vicié (102), et le deuxième raccord (31) du corps est relié à la deuxième conduite (103).

10. Dispositif d'assistance respiratoire selon la revendication 9, dans lequel la conduite d'air frais (101) et la conduite d'air vicié (102) sont formées chacune par un tuyau flexible séparé, qui sont couplés à la première (11) et deuxième (12) tubulure de raccordement correspondantes du premier raccord (10) d'un nébuliseur comportant les caractéristiques de la revendication 3, et l'appareil d'assistance respiratoire (100) est configuré de façon à pouvoir produire une circulation de base continue qui, pendant le cycle d'inspiration, circule conjointement avec l'air de ventilation depuis la conduite d'air frais (101) à travers le canal de circulation du corps (1, 2) vers la deuxième conduite (103) menant vers le patient (104), et, dans un autre cas, peut circuler vers la conduite d'air vicié (102), via la conduite d'air frais (101) et le canal de circulation secondaire (13) d'un nébuliseur avec les caractéristiques de la revendication 2.

11. Dispositif d'assistance respiratoire selon la revendication 9, dans lequel la conduite d'air frais (101) et la conduite d'air vicié (102) sont formées par un tuyau flexible commun, qui est divisé en deux parties par une cloison ou sous la forme d'un tuyau flexible coaxial et qui est couplé au premier raccord (10) du nébuliseur, et l'appareil d'assistance respiratoire (100) est configuré de façon à pouvoir produire une circulation de base continue qui, pendant le cycle d'inspiration, circule conjointement avec l'air de ventilation depuis la conduite d'air frais (101) à travers le canal de circulation du corps (1, 2) vers la deuxième conduite (103) menant vers le patient (104) et, dans un autre cas, peut circuler vers la conduite d'air vicié (103) en passant par la conduite d'air frais (101) et le canal de circulation secondaire (13) d'un nébuliseur avec les caractéristiques de la revendication 2, qui est formé entre la face frontale de la cloison, orientée vers le dispositif de nébulisation, ou le tuyau flexible intérieur du tuyau flexible coaxial, et le dispositif de nébulisation.

12. Dispositif d'assistance respiratoire selon l'une quelconque des revendications 8 à 11, comportant en outre une commande, par laquelle le dispositif de nébulisation (3) peut être déclenché conformément au cycle d'inspiration, de telle sorte qu'une nébulisation du fluide ne peut être activée que pendant le cycle d'inspiration.
